# EUROPÄISCHE PATENTSCHRIFT

(11) **EP 1 194 117 B1**
(45) Veröffentlichungstag und Bekanntmachung des Hinweises auf die Patenterteilung: **01.06.2005**
(21) Anmeldenummer: 00947919.7
(22) Anmeldetag: 04.07.2000
(51) Int. Cl.: A61K 7/13

(54) **MITTEL ZUM FÄRBEN VON KERATINHALTIGEN FASERN**
AGENT FOR DYEING KERATIN CONTAINING FIBRES
SUBSTANCE DESTINEE A LA TEINTURE DE FIBRES KERATINIQUES

(30) Priorität: 13.07.1999 DE 19932565
(43) Veröffentlichungstag der Anmeldung: 10.04.2002
(73) Patentinhaber: Henkel Kommanditgesellschaft auf Aktien, 40589 Düsseldorf (DE)
(72) Erfinder: MÖLLER, Hinrich, D-40789 Monheim (DE); HÖFFKES, Horst, D-40595 Düsseldorf (DE); OBERKOBUSCH, Doris, D-40591 Düsseldorf (DE)
(86) Internationale Anmeldenummer: PCT/EP2000/006220
(87) Internationale Veröffentlichungsnummer: WO 2001/003651

(56) Entgegenhaltungen:
- GB-A- 926 975
- US-A- 3 516 778
- US-A- 5 053 053
- US-A- 5 180 400

## Beschreibung

Die Erfindung betrifft ein Mittel zum Färben von keratinhaltigen Fasern, insbesondere menschlichen Haaren, das N-Halogenimine in Kombination mit ausgewählten Verbindungen enthält, die Verwendung dieser Kombination als färbende Komponente in Haarfärbemitteln sowie ein Verfahren zum Färben von keratinhaltigen Fasern, insbesondere menschlichen Haaren.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxyoder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluß von Oxidationsmitteln wie z. B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

In den Druckschriften US-A-5 053 053 und US-A-5 180 400 werden Mittel zum Färben keratinhaltiger Fasern beschrieben, welche zwingend mindestens ein Derivat des Indols, beispielsweise 5,6-Dihydroxyindol oder Aminoindol-Derivate, in Kombination mit einem Chinonderivat enthalten. Als Chinonderivate eignen sich beispielsweise p-Benzochinon, 1,4-Naphthochinon sowie deren Mono- oder Diimin-Derivate mit halogeniertem Imin-Stickstoffatom. Das Chinonderivat fungiert effektiv als Oxidationsmittel für das Indolderivat. Das Redox-Potenzial der Indolderivate und der Chinonderivate muß eine definierte Potenzialdifferenz besitzen.

Die Verwendung der unten näher beschriebenen Mittel zum Färben von keratinhaltigen Fasern ist bislang nicht bekannt.

Aufgabe der vorliegenden Erfindung ist es, Färbemittel für Keratinfasern, insbesondere menschliche Haare, bereitzustellen, die hinsichtlich der Farbtiefe, der Grauabdeckung und den Echtheitseigenschaften qualitativ den üblichen Oxidationshaarfärbemitteln mindestens gleichwertig sind, ohne jedoch unbedingt auf Oxidationsmittel wie z. B. H₂O₂ angewiesen zu sein. Darüber hinaus dürfen die Färbemittel kein oder lediglich ein sehr geringes Sensibilisierungspotential aufweisen.

Überraschenderweise wurde nun gefunden, daß die erfindungsgemäßen Mittel sich auch in Abwesenheit von oxidierenden Agentien hervorragend zum Färben von keratinhaltigen Fasern eignen. Sie ergeben Ausfärbungen mit hervorragender Brillanz und Farbtiefe und führen zu vielfältigen Farbnuancen. Der Einsatz von oxidierenden Agentien soll dabei jedoch nicht prinzipiell ausgeschlossen- werden.

Gegenstand der Erfindung ist ein Mittel zum Färben von keratinhaltigen Fasern, insbesondere menschlichen Haaren, enthaltend N-Halogenimine mit der Formel I, 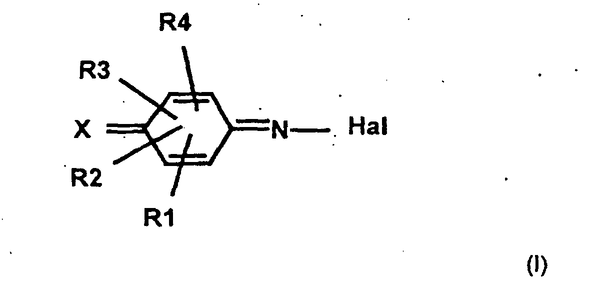 in der
- R¹, R², R³, R⁴: für ein Wasserstoffatom, ein Halogenatom, eine C₁-C₄-Alkylgruppe, eine C₁-C₄-Alkoxygruppe, wobei auch zwei der Reste gemeinsam einen ankondensierten aromatischen bzw. heteroaromatischen Ring bilden können, stehen,
- X: für Sauerstoff oder ein Arylimin steht und
- Hal: für Fluor, Chlor, Brom oder Jod steht
und zusätzlich als Komponente B mindestens eine Verbindung ausgewählt aus mindestens einer CH-aktiven Verbindung und/oder mindestens einer Verbindung aus der Gruppe, die gebildet wird aus
m-Phenylendiamin, 2,4-Diaminophenoxyethanol, 3-Amino-2,4-dichlor-, 2-Methyl-5-amino-, 2-Methyl-5-(2-hydroxyethylamino)-, 2-Methyl-5-amino-4-chlor-, 6-Methyl-3-amino-2-chlor-phenol, 4-Aminobenzoesäure, 3,5-Diaminobenzoesäure, 5-Aminosalicylsäure, 3-Amino-4-hydroxy-, 4-Amino-3-hydroxy-benzoesäure, 4-Aminobenzolsulfonsäure, 3-Amino-4-hydroxybenzolsulfonsäure, 4-Amino-3-hydroxynaphthalin-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 4,5-Diaminobrenzcatechinsulfat, 3,5-Diamino-4-hydroxybrenzcatechin-sulfat, 4-Nitroanilin, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 2-Amino-6-chlor-4-nitrophenol, 1-Amino-5-chlor-4-(2-hydroyethylamino)-2-nitrobenzol, 4,4'-Diaminostilben-dihydrochlorid, 4,4'-Diaminostilben-2,2'-disulfonsäure, Na-Salz, 4,4'-Diaminodiphenylmethan, 4,4'-Diaminobenzophenon, 3,3',4,4'-Tetraaminodiphenyl-tetrahydrochlorid, 1,3-Bis-(2,4-diaminophenoxy)-propan-tetrahydrochlorid und 1,3-Bis-[N-(4-aminophenyl)-2-hydroxyethylamino]-2-propanol, 4-Amino-, 2-Amino-3-hydroxy-, 3-Amino-2-methylamino-6-methoxy-, 2,6-Dihydroxy-3,4-dimethylpyridin, 2,3,4-Trimethylpyrrol, 2,4-Dimethyl-3-ethylpyrrol, 8-Aminochinolin, 6-Aminonicotinsäure, 5-Aminoisochinolin, 7-Amino-benzimidazol und 2,5-Dihydroxy-4-morpholinoanilin sowie jeweils aus den mit vorzugsweise anorganischen Säuren gebildeten physiologisch verträglichen Salzen dieser Verbindungen, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 4-Methoxy-, 3-Dimethylamino-, 2-(2-Hydroxyethyl)-, 3,4-Methylendioxyphenol, 3,4-Dihydroxybenzoesäure, Gallussäure, 2,4,6-Trihydroxybenzoesäure, 4-Chlorresorcin, 1-Naphthol, 2,7-Dihydroxynaphthalin, 6-Dimethylamino-4-hydroxy-2-naphthalinsulfonsäure und 3,6-Dihydroxy-2,7-naphthalinsulfonsäure.

Unter keratinhaltigen Fasern sind Wolle, Pelze, Federn und insbesondere menschliche Haare zu verstehen.

Vorzugsweise sind die Verbindungen mit der Formel I ausgewählt aus der Gruppe folgender N-Halogenimine: N-Chlor-p-benzochinonimin, 2,6-Dichlor-p-benzochinon-4-chlorimin, 2,6-Dibrom-p-benzochinon-4-chlorimin, N-chlor-N'-phenyl-pbenzochinondiimin sowie deren beliebigen Gemische.

Diese Substanzen sind zum großen Teil literaturbekannt oder im Handel erhältlich oder nach bekannten Syntheseverfahren herstellbar.

Die voranstehend genannten N-Halogenimine mit der Formel I werden vorzugsweise in den erfindungsgemäßen Mitteln in einer Menge von 0,03 bis 65 mmol, insbesondere von 1 bis 40 mmol, bezogen auf 100 g des gesamten Färbemittels; verwendet. Sie können als direktziehende Färbemittel oder in Gegenwart von üblichen Oxidationsfarbstoffvorprodukten eingesetzt werden.

Mit den erfindungsgemäßen Färbemitteln werden Färbungen mit erhöhter Brillanz und verbesserten Echtheitseigenschaften (Lichtechtheit, Waschechtheit, Reibechtheit) über einen weiten Nuancenbereich von gelb über gelbbraun, orange, braunorange, mittelbraun, olivgrün, dunkelbraun, violett, dunkelviolett bis hin zu blauschwarz und schwarz erzielt.

Die erfindungsgemäßen Verbindungen der Komponente B können in einer Menge von jeweils 0,03 bis 65, insbesondere 1 bis 40 mmol, jeweils bezogen auf 100 g des gesamten Färbemittels, eingesetzt werden.

In allen Färbemitteln können auch mehrere verschiedene N-Halogenimine der Formel I gemeinsam zum Einsatz kommen; ebenso können auch mehrere verschiedene Verbindungen der Komponente B gemeinsam verwendet werden.

Unter die voranstehend beschriebene Ausführungsform fällt auch die Verwendung von solchen Substanzen, die Reaktionsprodukte von N-Halogeniminen der Formel I mit den genannten Verbindungen der Komponente B darstellen, als direktziehende Färbemittel. Derartige Reaktionsprodukte können z. B. durch kurzes Erwärmen der beiden Komponenten in stöchiometrischen Mengen in wäßrigem neutralen. bis schwach alkalischen Milieu erhalten werden, wobei sie entweder als Feststoff aus der Lösung ausfallen oder durch Eindampfen der Lösung daraus isoliert werden. Die Reaktionsprodukte können auch in Kombination mit anderen Farbstoffen oder Farbstoffvorprodukten eingesetzt werden.

Als CH-aktive Verbindungen können beispielhaft genannt werden 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, Fischersche Base (1,3,3-Trimethyl-2-methylenindolin), 2,3-Dimethyl-benzothiazoliumiodid, 2,3-Dimethyl-benzothiazolium-p-toluolsulfonat, Rhodanin, Rhodanin-3-essigsäure, 1-Methyl-2-chinaldinium-iodid, 1-Ethyl-2-chinaldinium-iodid, 1,4-Dimethylchinolinium-iodid, Barbitursäure, Thiobarbitursäure, 1,3-Dimethylthiobarbitursäure, 1,3-Diethylthiobarbitursäure, Diethylthiobarbitursäure, Oxindol, 3-Indoxylacetat, Cumaranon und 1-Methyl-3-phenyl-2-pyrazolinon.

Die Verbindungen der Komponente B werden besonders bevorzugt ausgewählt aus der Gruppe bestehend aus 2-(2,4-Diaminophenoxy)-ethanol, 3-Amino-2,4-dichlor-phenol, 2-Methyl-5-amino-phenol, 2-Methyl-5-(2-hydroxyethylamino)-phenol, 2-Methyl-5-amino-4-chlor-phenol, 6-Methyl-3-amino-2-chlor-phenol, 3,4-Diaminobenzoesäure, 3-Amino-2-methylamino-6-methoxypyridin und 2,6-Dihydroxy-3,4-dimethylpyridin, sowie jeweils aus den vorzugsweise mit anorganischen Säuren gebildeten physiologisch verträglichen Salzen dieser Verbindungen.

Auf die Anwesenheit von Oxidationsmitteln, z. B. H₂O₂, kann dabei verzichtet werden. Es kann jedoch u. U.. wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z. B. aus der Gruppe der Nitrophenylendiamine, Nitroaminophenole, Anthrachinone oder Indophenole, wie z. B. die unter den internationalen Bezeichnungen bzw. Handelsnamen HC Yellow 2, HC Yellow 4, HC Yellow 6, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16 und Basic Brown 17 bekannten Verbindungen sowie Pikraminsäure 2-Amino-6-chloro-4-nitrophenol, 4-Amino-2-nitrodiphenylamin-2'carbonsäure, 6-Nitro-1,2,3,4-tetrahydrochinoxalin, 4-N-Ethyl-1,4-bis(2'-hydroxyethylamino)-2-nitrobenzol-hydrochlorid und 1-Methyl-3-nitro-4-(2'-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die Oxidationsfarbstoffvorprodukte oder die fakultativ enthaltenen direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Färbemittel inwasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykoloder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobemsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quatemisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Coming 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquatemäre Polydimethylsiloxane, Quatemium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quatemisierte Celluloseether, Polysiloxane mit quatemären Gruppen, Dimethyldiallylammoniumchlorid-Polymere, Acrylamid-Dimethyldiallylammoniumchlorid-Copolymere, mit Diethylsulfat quatemierte Dimethylaminoethylmethacrylat-Vinylpyrrolidon-Copolymere, Vinylpyrrolidon-Imidazoliniummethochlorid-Copolymere und quatemierter Polyvinylalkohol,
- zwitterionische und amphotere Polymere wie beispielsweise Acrylamidopropyltrimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vemetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40, mmol bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 9.

Ein weiterer Gegenstand der vorliegenden Erfindung betrifft die Verwendung von N-Halogeniminen mit der Formel I, 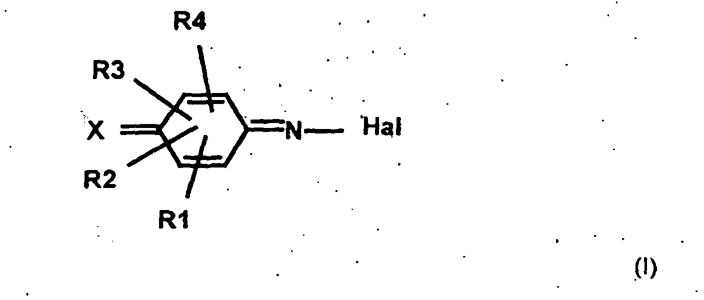 in der
- R¹, R², R³, R⁴: für ein Wasserstoffatom, ein Halogenatom, eine C₁-C₄-Alkylgruppe, eine C₁-C₄-Alkoxygruppe, wobei auch zwei der Reste gemeinsam einen ankondensierten aromatischen bzw. heteroaromatischen Ring bilden können, stehen,
- X: für Sauerstoff oder ein Arylimin steht und
- Hal: für Fluor, Chlor, Brom oder Jod steht,
in Kombination mit mindestens einer Verbindung ausgewählt aus mindestens einer CH-aktiven Verbindung und/oder mindestens einer Verbindung aus der Gruppe, die gebildet wird aus
m-Phenylendiamin, 2,4-Diaminophenoxyethanol, 3-Amino-2,4-dichlor-, 2-Methyl-5-amino-, 2-Methyl-5-(2-hydroxyethylamino)-, 2-Methyl-5-amino-4-chlor-, 6-Methyl-3-amino-2-chlor-phenol, 4-Aminobenzoesäure, 3,5-Diaminobenzoesäure, 5-Aminosalicylsäure, 3-Amino-4-hydroxy-, 4-Amino-3-hydroxy-benzoesäure, 4-Aminobenzolsulfonsäure, 3-Amino-4-hydroxybenzolsulfonsäure, 4-Amino-3-hydroxynaphthalin-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 4,5-Diaminobrenzcatechinsulfat, 3,5-Diamino-4-hydroxybrenzcatechin-sulfat, 4-Nitroanilin, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 2-Amino-6-chlor-4-nitrophenol, 1-Amino-5-chlor-4-(2-hydroyethylamino)-2-nitrobenzol, 4,4'-Diaminostilben-dihydrochlorid, 4,4'-Diaminostilben-2,2'-disulfonsäure, Na-Salz, 4,4'-Diaminodiphenylmethan, 4,4'-Diaminobenzophenon, 3,3',4,4'-Tetraaminodiphenyl-tetrahydrochlorid, 1,3-Bis-(2,4-diaminophenoxy)-propan-tetrahydrochlorid und 1,3-Bis-[N-(4-aminophenyl)-2-hydroxyethylamino]-2-propanol, 4-Amino-, 2-Amino-3-hydroxy-, 3-Amino-2-methylamino-6-methoxy-, 2,6-Dihydroxy-3,4-dimethylpyridin, 2,3,4-Trimethylpyrrol, 2,4-Dimethyl-3-ethylpyrrol, 8-Aminochinolin, 6-Aminonicotinsäure, 5-Aminoisochinolin, 7-Amino-benzimidazol und 2,5-Dihydroxy-4-morpholinoanilin sowie jeweils aus den mit vorzugsweise anorganischen Säuren gebildeten physiologisch verträglichen Salzen dieser Verbindungen, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 4-Methoxy-, 3-Dimethylamino-, 2-(2-Hydroxyethyl)-, 3,4-Methylendioxyphenol, 3,4-Dihydroxybenzoesäure, Gallussäure, 2,4,6-Trihydroxybenzoesäure, 4-Chlorresorcin, 1-Naphthol, 2,7-Dihydroxynaphthalin, 6-Dimethylamino-4-hydroxy-2-naphthalinsulfonsäure und 3,6-Dihydroxy-2,7-naphthalinsulfonsäure, als eine färbende Komponente in Oxidationshaarfärbemitteln.

Noch ein weiterer Gegenstand der vorliegenden Erfindung betrifft ein Verfahren zum Färben von keratinhaltigen Fasern, insbesondere menschlichen Haaren, worin ein Färbemittel, enthaltend,
A) mindestens ein N-Halogenimin mit der Formel I, 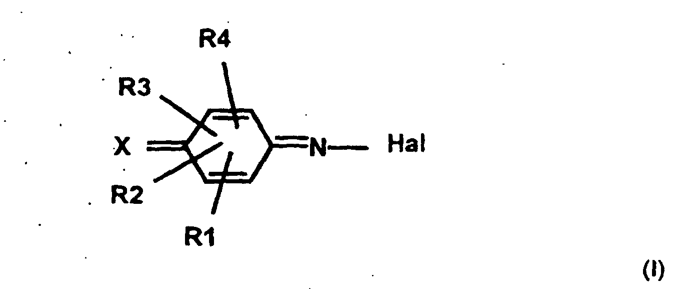 in der
   - R¹, R², R³, R⁴: für ein Wasserstoffatom, ein Halogenatom, eine C₁-C₄-Alkylgruppe, eine C₁-C₄-Alkoxygruppe, wobei auch zwei der Reste gemeinsam einen ankondensierten aromatischen bzw. heteroaromatischen Ring bilden können, stehen,
   - X: für Sauerstoff oder ein Arylimin steht und
   - Hal: für Fluor, 'Chlor, Brom oder Jod steht
   und
B) mindestens eine CH-aktive Verbindung und/oder mindestens eine Verbindung aus der Gruppe, die gebildet wird aus
   m-Phenylendiamin, 2,4-Diaminophenoxyethanol, 3-Amino-2,4-dichlor-, 2-Methyl-5-amino-, 2-Methyl-5-(2-hydroxyethylamino)-, 2-Methyl-5-amino-4-chlor-, 6-Methyl-3-amino-2-chlor-phenol, 4-Aminobenzoesäure, 3,5-Diaminobenzoesäure, 5-Aminosalicylsäure, 3-Amino-4-hydroxy-, 4-Amino-3-hydroxy-benzoesäure, 4-Aminobenzolsulfonsäure, 3-Amino-4-hydroxybenzolsulfonsäure, 4-Amino-3-hydroxynaphthalin-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 4,5-Diaminobrenzcatechinsulfat, 3,5-Diamino-4-hydroxybrenzcatechin-sulfat, 4-Nitroanilin, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 2-Amino-6-chlor-4-nitrophenol, 1-Amino-5-chlor-4-(2-hydroyethylamino)-2-nitrobenzol, 4,4'-Diaminostilben-dihydrochlorid, 4,4'-Diaminostilben-2,2'disulfonsäure, Na-Salz, 4,4-Diaminodiphenylmethan, 4,4'-Diaminobenzophenon, 3,3',4,4'-Tetraaminodiphenyl-tetrahydrochlorid, 1,3-Bis-(2,4-diaminophenoxy)-propan-tetrahydrochlorid und 1,3-Bis-[N-(4-aminophenyl)-2-hydroxyethylamino]-2-propanol,
   4-Amino-, 2-Amino-3-hydroxy-, 3-Amino-2-methylamino-6-methoxy-, 2,6-Dihydroxy-3,4-dimethylpyridin, 2,3,4-Trimethylpyrrol, 2,4-Dimethyl-3-ethyl-pyrrol, 8-Aminochinolin, 6-Aminonicotinsäure, 5-Aminoisochinolin, 7-Amino-benzimidazol und 2,5-Dihydroxy-4-morpholinoanilin sowie jeweils aus den mit vorzugsweise anorganischen Säuren gebildeten physiologisch verträglichen Salzen dieser Verbindungen,
   5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 4-Methoxy-, 3-Dimethylamino-, 2-(2-Hydroxyethyl)-, 3,4-Methylendioxyphenol, 3,4-Dihydroxybenzoesäure, Gallussäure, 2,4,6-Trihydroxybenzoesäure, 4-Chlorresorcin, 1-Naphthol, 2,7-Dihydroxynaphthalin, 6-Dimethylamino-4-hydroxy-2-naphthalinsulfonsäure und 3,6-Dihydroxy-2,7-naphthalinsulfonsäure.
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

Die N-Halogenimine der Formel I und die Verbindungen der Komponente B können entweder gleichzeitig auf das Haar aufgebracht werden oder aber auch nacheinander, wobei es unerheblich ist, welche der beiden Komponenten zuerst aufgetragen wird. Die fakultativ enthaltenen Ammonium- oder Metallsalze können dabei der ersten oder der zweiten Komponente zugesetzt werden. Zwischen dem Auftragen der ersten und der zweiten Komponente können bis zu 30 Minuten Zeitabstand liegen. Auch eine Vorbehandlung der Fasern mit der Salzlösung ist möglich.

Die N-Halogenimine der Formel I und die Verbindungen der Komponente B können entweder getrennt oder zusammen gelagert werden, entweder in einer flüssigen bis pastösen Zubereitung (wäßrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (30 bis 80°C) Wassers hinzugefügt und eine homogene Mischung hergestellt.

### Beispiele

### Herstellung einer Färbelösung

Es wurde je eine Aufschlämmung von 5 mmol eines N-Halogenimins mit der Formel I, 5 mmol eines Oxidationsfarbstoffvorproduktes der Komponente B und 5 mmol Natriumacetat und einen Tropfen einer 20-%igen Fettalkylethersulfat-Lösung in 50 ml Wasser bei 60°C hergestellt. Die Aufschlämmungen wurden nach Abkühlen auf 30°C miteinander vermischt und mit verdünnter NaOH oder Salzsäure auf einen pH-Wert von 6 eingestellt.

In diese Färbemischung wurde bei 30°C 30 Minuten lang eine Strähne zu 90% ergrauten, nicht vorbehandelten Menschenhaares eingebracht. Die Strähne wurde dann 30 Sek. mit lauwarmem Wasser gespüft, mit warmer Luft getrocknet und anschließend ausgekämmt.

Die jeweiligen Farbnuancen und Farbtiefen sind in der nachfolgenden. Tabelle 1 wiedergegeben.

Die Farbtiefe wurde dabei nach folgender Skala bewertet:

| | |
|---|---|
| - | keine oder eine sehr blasse Ausfärbung |
| (+) | schwache Intensität |
| + | mittlere Intensität |
| +(+) | mittlere bis starke Intensität |
| ++ | starke Intensität |
| ++(+) | starke bis sehr starke Intensität |
| +++ | sehr starke Intensität |

**Tabelle 1**

| Ausfärbungen mit N-Chlor-p-benzochinonimin | | | |
|---|---|---|---|
| **Komponente B** | **pH-Wert** | **Farbe** | **Intensität** |
| Gallussäure | 9,0 | mittelbraun | +(+) |
| 2,7-Dihydroxynaphthalin | 9,0 | olivgrün | + |
| 1-Naphthol | 9,0 | hellbraun | + |
| 1-Hydroxy-3-amino-6-methylbenzol | 9,0 | schwarz | +++ |
| 2-Chlor-6-methyl-3-amino-phenol x HCl | 9,0 | braunorange | ++ |

**Tabelle 2**

| Ausfärbungen mit 2,6-Dichlorchinon-4-chlorimin | | | |
|---|---|---|---|
| **Komponente B** | **pH-Wert** | **Farbe** | **Intensität** |
| Gallussäure | 6,0 | hellbraun | + |
| 2,7-Dihydroxynaphthalin | 6,0 | silbergrau | + |
| 1-Naphthol | 6,0 | hellgrau-violett | + |
| 1-Hydroxy-3-amino-6-methylbenzol | 6,0 | orangebraun | +(+) |
| 2-Chlor-6-methyl-3-amino-phenol x HCl | 6,0 | grau | +(+) |

## Patentansprüche

1. Mittel zum Färben von keratinhältigen Fasern, insbesondere menschlichen Haaren, enthaltend als färbende Komponenten mindestens ein N-Halogenimin mit der Formel I, 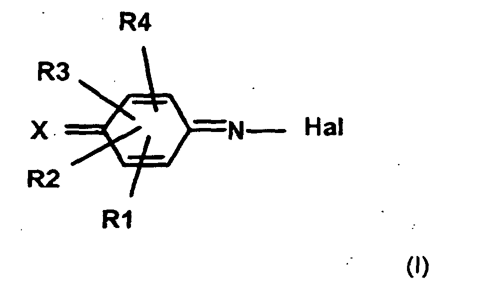 in der
R¹, R², R³, R⁴ für ein Wasserstoffatom, ein Halogenatom, eine C₁-C₄-Alkylgruppe, eine C₁-C₄-Alkoxygruppe, wobei auch zwei der Reste gemeinsam einen ankondensierten aromatischen bzw. heteroaromatischen Ring bilden können, stehen,
X für Sauerstoff oder ein Arylimin steht und
Hal für Fluor, Chlor, Brom oder Jod steht
und zusätzlich mindestens eine Verbindung ausgewählt aus mindestens einer CH-aktiven Verbindung und/oder mindestens einer Verbindung aus der Gruppe, die gebildet wird aus
m-Phenylendiamin, 2,4-Diaminophenoxyethanol, 3-Amino-2,4-dichlor-, 2-Methyl-5-amino-, 2-Methyl-5-(2-hydroxyethylamino)-, 2-Methyl-5-amino-4-chlor-, 6-Methyl-3-amino-2-chlor-phenol, 4-Aminobenzoesäure, 3,5-Diaminobenzoesäure, 5-Aminosalicylsäure, 3-Amino-4-hydroxy-, 4-Amino-3-hydroxy-benzoesäure, 4-Aminobenzolsulfonsäure, 3-Amino-4-hydroxybenzolsulfonsäure, 4-Amino-3-hydroxynaphthalin-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 4,5-Diaminobrenzcatechinsulfat, 3,5-Diamino-4-hydroxybrenzcatechin-sulfat, 4-Nitroanilin, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 2-Amino-6-chlor-4-nitrophenol, 1-Amino-5-chlor-4-(2-hydroyethylamino)-2-nitrobenzol, 4,4'-Diaminostilben-dihydrochlorid, 4,4'-Diaminostilben-2,2'-disulfonsäure, Na-Salz, 4,4'-Diaminodiphenylmethan, 4,4'-Diaminobenzophenon, 3,3',4,4'-Tetraaminodiphenyl-tetrahydrochlorid, 1,3-Bis-(2,4-diaminophenoxy)-propantetrahydrochlorid und 1,3-Bis-[N-(4-aminophenyl)-2-hydroxyethylamino]-2-propanol, 4-Amino-, 2-Amino-3-hydroxy-, 3-Amino-2-methylamino-6-methoxy-, 2,6-Dihydroxy-3,4-dimethylpyridin, 2,3,4-Trimethylpyrrol, 2,4-Dimethyl-3-ethyl-pyrrol, 8-Aminochinolin, 6-Aminonicotinsäure, 5-Aminoisochinolin, 7-Amino-benzimidazol und 2,5-Dihydroxy-4-morpholinoanilin sowie jeweils aus den mit vorzugsweise anorganischen Säuren gebildeten physiologisch verträglichen Salzen dieser Verbindungen,
5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 4-Methoxy-, 3-Dimethylamino-, 2-(2-Hydroxyethyl)-, 3,4-Methylendioxyphenol, 3,4-Dihydroxybenzoesäure, Gallussäure, 2,4,6-Trihydroxybenzoesäure, 4-Chlorresorcin, 1-Naphthol, 2,7-Dihydroxynaphthalin, 6-Dimethylamino-4-hydroxy-2-naphthalinsulfonsäure und 3,6-Dihydroxy-2,7-naphthalinsulfonsäure.

2. Mittel nach Anspruch 1, **dadurch gekennzeichnet, daß** als Verbindungen mit der Formel I N-Chlor-p-benzochinonimin, 2,6-Dichlor-p-benzochinon-4-chlorimin, 2,6-Dibrom-p-benzochinon-4-chlorimin, N-chlor-N'-phenyl-p-benzochinondiimin sowie deren beliebigen Gemische eingesetzt werden.

3. Mittel nach einem der Ansprüche 1 bis 2, **dadurch gekennzeichnet, daß** die N-Halogenimine mit der Formel I in einer Menge von 0,03 bis 65 mmol, insbesondere von 1 bis 40 mmol, bezogen auf 100 g des gesamten Färbemittels, enthalten sind.

4. Mittel nach einem der Ansprüche 1 bis 3, **dadurch gekennzeichnet, daß** die CH-aktive Verbindung ausgewählt wird aus der Gruppe, bestehend aus 1,2,3,3-Tetramethyl-3H-indoliumiodid, 1,2,3,3-Tetramethyl-3H-indolium-p-toluotsulfonat, 1,2,3,3-Tetramethyl-3H-indolium-methansulfonat, Fischersche Base (1,3,3-Trimethyl-2-methylenindolin), 2,3-Dimethyl-benzothiazoliumiodid, 2,3-Dimethyl-benzothiazolium-p-toluolsulfonat, Rhodanin, Rhodanin-3-essigsäure, 1-Ethyl-2-chinaldinium-iodid, 1,4-Dimethylchinolinium-iodid, 1-Methyl-2-chinaldinium-iodid, 1,4-Dimethylchinolinium-iodid, Barbitursäure, Thiobarbitursäure, 1,3-Dimethylthiobarbitursäure, 1,3-Diethylthiobarbitursäure, Diethylthiobarbitursäure, Oxindol, 3-Indoxylacetat, Cumaranon und 1-Methyl-3-phenyl-2-pyrazolinon.

5. Mittel nach einem der Ansprüche 1 bis 4, **dadurch gekennzeichnet, daß** die weitere Verbindung ausgewählt ist aus der Gruppe bestehend aus 2-(2,4-Diaminophenoxy)-ethanol; 3-Amino-2,4-dichlor-phenol, 2-Methyl-5-amino-phenol, 2-Methyl-5-(2-hydroxyethylamino)-phenol, 2-Methyl-5-amino-4-chlor-phenol, 6-Methyl-3-amino-2-chlor-phenol, 3,4-Diaminobenzoesäure, 3-Amino-2-methylamino-6-methoxy-pyridin und 2,6-Dihydroxy-3,4-dimethylpyridin, sowie jeweils aus den vorzugsweise mit anorganischen Säuren gebildeten physiologisch verträglichen Salzen dieser Verbindungen.

6. Mittel nach einem der Ansprüche 1 bis 5, **dadurch gekennzeichnet, daß** es direkt ziehende Farbstoffe aus der Gruppe der Nitrophenylendiamine, Nitroaminophenole, Anthrachinone oder Indophenole vorzugsweise in einer Menge von 0,01 bis 20 Gew.-%, bezogen auf das gesamte Färbemittel, enthält.

7. Mittel nach einem der Ansprüche 1 bis 6, **dadurch gekennzeichnet, daß** Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, zusätzlich enthalten sind.

8. Mittel nach einem der Ansprüche 1 bis 7, **dadurch gekennzeichnet, daß** es Oxidationsmittel, insbesondere H₂O₂, in einer Menge von 0,01 bis 6 Gew.-%, bezogen auf die Anwendungslösung, enthält.

9. Mittel nach einem der Ansprüche 1 bis 8, **dadurch gekennzeichnet, daß** es anionische, zwitterionische oder nichtionische Tenside enthält.

10. Verfahren zum Färben von keratinhaltigen Fasern, insbesondere menschlichen Haaren, worin ein Färbemittel, enthaltend
A) mindestens ein N-Halogenimin mit der Formel I, 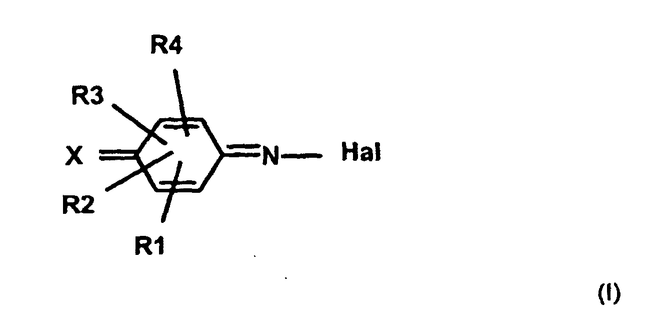 in der
R¹, R², R³, R⁴ für ein Wasserstoffatom, ein Halogenatom, eine C₁-C₄-Alkylgruppe, eine C₁-C₄-Alkoxygruppe, wobei auch zwei der Reste gemeinsam einen ankondensierten aromatischen bzw. heteroaromatischen Ring bilden können, stehen,
X für Sauerstoff oder ein Arylimin steht und
Hal für Fluor, Chlor, Brom oder Jod steht, und
B) mindestens eine CH-aktive Verbindung und/oder mindestens eine Verbindung aus der Gruppe, die gebildet wird aus
m-Phenylendiamin, 2,4-Diaminophenoxyethanol, 3-Amino-2,4-dichlor-, 2-Methyl-5-amino-, 2-Methyl-5-(2-hydroxyethylamino)-, 2-Methyl-5-amino-4-chlor-, 6-Methyl-3-amino-2-chlor-phenol, 4-Aminobenzoesäure, 3,5-Diaminobenzoesäure, 5-Aminosalicylsäure, 3-Amino-4-hydroxy-, 4-Amino-3-hydroxy-benzoesäure, 4-Aminobenzolsulfonsäure, 3-Amino-4-hydroxybenzolsulfonsäure, 4-Amino-3-hydroxynaphthalin-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 4,5-Diaminobrenzcatechinsulfat, 3,5-Diamino-4-hydroxybrenzcatechin-sulfat, 4-Nitroanilin, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 2-Amino-6-chlor-4-nitrophenol, 1-Amino-5-chlor-4-(2-hydroyethylamino)-2-nitrobenzol, 4,4'-Diaminostilben-dihydrochlorid, 4,4'-Diaminostilben-2,2'disulfonsäure, Na-Salz, 4,4'-Diaminodiphenylmethan, 4,4'-Diaminobenzophenon, 3,3',4,4'-Tetraaminodiphenyl-tetrahydrochlorid, 1,3-Bis-(2,4-diaminophenoxy)-propan-tetrahydrochlorid und 1,3-Bis-[N-(4-aminophenyl)-2-hydroxyethylamino]-2-propanol,
4-Amino-, 2-Amino-3-hydroxy-, 3-Amino-2-methylamino-6-methoxy-, 2,6-Dihydroxy-3,4-dimethylpyridin, 2,3,4-Trimethylpyrrol, 2,4-Dimethyl-3-ethylpyrrol, 8-Aminochinolin, 6-Aminonicotinsäure, 5-Aminoisochinolin, 7-Amino-benzimidazol und 2,5-Dihydroxy-4-morpholinoanilin sowie jeweils aus den mit vorzugsweise anorganischen Säuren gebildeten physiologisch verträglichen Salzen dieser Verbindungen,
5-Methylresorcin, 2,5-Dimethylresorcin, Resorcin, 3-Methoxyphenol, Brenzkatechin, Hydrochinon, Pyrogallol, Phloroglucin, Hydroxyhydrochinori, 4-Methoxy-, 3-Dimethylamino-, 2-(2-Hydroxyethyl)-, 3,4-Methylendioxyphenol, 3,4-Dihydroxybenzoesäure, Gallussäure, 2,4,6-Trihydroxybenzoesäure, 4-Chlorresorcin, 1-Naphthol, 2,7-Dihydroxynaphthalin, 6-Dimethylamino-4-hydroxy-2-naphthalinsulfonsäure und 3,6-Dihydroxy-2,7-naphthalinsulfonsäure.
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Claims

1. Preparation for colouring keratin-containing fibres, more particularly human hair, which contain as colouring components at least one N-haloimine corresponding to formula I: 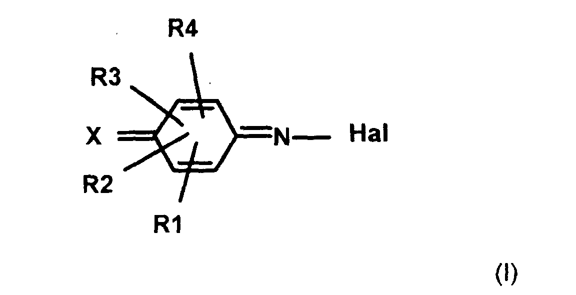 in which R¹, R², R³ and R⁴ represent a hydrogen atom, a halogen atom, a C₁₋₄ alkyl group, a C₁₋₄ alkoxy group; two of the substituents together may even form a fused aromatic or heteroaromatic ring,
X is oxygen or an arylimine and
Hal is fluorine, chlorine, bromine or iodine,
and, in addition, at least one compound selected from at least one CH-ative compound and/or at least one compound from the group consisting of m-phenylenediamine, 2,4-diaminophenoxyethanol, 3-amino-2,4-dichloro-, 2-methyl-5-amino-, 2-methyl-5-(2-hydroxyethylamino)-, 2-methyl-5-amino-4-chloro-, 6-methyl-3-amino-2-chlorophenol, 4-aminobenzoic acid, 3,5-diaminobenzoic acid, 5-aminosalicylic acid, 3-amino-4-hydroxy-, 4-amino-3-hydroxybenzoic acid, 4-aminobenzenesulfonic acid, 3-amino-4-hydroxybenzenesulfonic acid, 4-amino-3-hydroxynaphthalenesulfonic acid, 6-amino-7-hydroxynaphthalene-2-sulfonic acid, 7-amino-4-hydroxynaphthalene-2-sulfonic acid, 4-amino-5-hydroxynaphthalene-2,7-disulfonic acid, 3-amino-2-naphthoic acid, 3-aminophthalic acid, 5-aminoisophthalic acid, 4,5-diaminopyrocatechol sulfate, 3,5-diamino-4-hydroxypyrocatechol sulfate, 4-nitroaniline, 4-nitro-1,3-phenylenediamine, 2-nitro-4-amino-1-(2-hydroxyethylamino)-benzene, 2-nitro-1-amino-4-[bis-(2-hydroxyethyl)-amino]-benzene, 4-amino-2-nitrodiphenylamine-2'-carboxylic acid, 2-amino-6-chloro-4-nitrophenol, 1-amino-5-chloro-4-(2-hydroyethylamino)-2-nitrobenzene, 4,4'-diaminostilbene dihydrochloride, 4,4'-diaminostilbene-2,2'-disulfonic acid, sodium salt, 4,4'-diaminodiphenylmethane, 4,4'-diaminobenzophenone, 3,3',4,4'-tetraaminodiphenyl tetrahydrochloride, 1,3-bis-(2,4-diaminophenoxy)-propane tetrahydrochloride and 1,3-bis-[N-(4-aminophenyl)-2-hydroxyethylamino]-2-propanol,
4-amino-, 2-amino-3-hydroxy-, 3-amino-2-methylamino-6-methoxy-, 2,6-dihydroxy-3,4-dimethylpyridine, 2,3,4-trimethylpyrrole, 2,4-dimethyl-3-ethylpyrrole, 8-aminoquinoline, 6-aminonicotinic acid, 5-aminoisoquinoline, 7-aminobenzimidazole and 2,5-dihydroxy-4-morpholinoaniline and physiologically safe salts of these compounds formed with preferably inorganic acids,
5-methylresorcinol, 2,5-dimethylresorcinol, resorcinol, 3-methoxyphenol, pyrocatechol, hydroquinone, pyrogallol, phloroglucinol, hydroxyhydroquinone, 4-methoxy-, 3-dimethylamino-, 2-(2-hydroxyethyl)-, 3,4-methylenedioxyphenol, 3,4-dihydroxybenzoic acid, gallic acid, 2,4,6-trihydroxybenzoic acid, 4-chlororesorcinol, 1-naphthol, 2,7-dihydroxynaphthalene, 6-dimethylamino-4-hydroxy-2-naphthalene sulfonic acid and 3,6-dihydroxy-2,7-naphthalenesulfonic acid.

2. Preparation as claimed in claim 1, **characterized in that** N-chloro-p-benzoquinone imine, 2,6-dichloro-p-benzoquinone-4-chloroimine, 2,6-di-bromo-p-benzoquinone-4-chloroimine, N-chloro-N'-phenyl-p-benzoquinone diimine and mixtures thereof are used as the compounds of formula I.

3. Preparation as claimed in any of claims 1 to 2, **characterized in that** the N-haloimines of formula I are present in a quantity of 0.03 to 65 mmol and more particularly in a quantity of 1 to 40 mmol, based on 100 g of the colorant as a whole.

4. Preparation as claimed in any of claims 1 to 3, **characterized in that** the CH-active compound is selected from the group consisting of 1,2,3,3-tetramethyl-3H-indolium iodide, 1,2,3,3-tetramethyl-3H-indolium-p-toluene-sulfonate, 1,2,3,3-tetramethyl-3H-indolium methanesulfonate, Fischer's base (1,3,3-trimethyl-2-methyleneindoline), 2,3-dimethylbenzothiazolium iodide, 2,3-dimethyl benzothiazolium-p-toluenesulfonate, rhodanine, rhodanine-3-acetic acid, 1-ethyl-2-quinaldinium iodide, 1,4-dimethylquinolinium iodide, 1-methyl-2-quinaldinium iodide, 1,4-dimethylquinolinium iodide, barbituric acid, thiobarbituric acid, 1,3-dimethylthiobarbituric acid, 1,3-diethylthiobarbituric acid, diethylthiobarbituric acid, oxindole, 3-indoxylacetate, coumaranone and 1-methyl-3-phenyl-2-pyrazolinone.

5. Preparation as claimed in any of claims 1 to 4, **characterized in that** the other compound is selected from the group consisting of 2-(2,4-diaminophenoxy)-ethanol, 3-amino-2,4-dichlorophenol, 2-methyl-5-aminophenol, 2-methyl-5-(2-hydroxyethylamino)-phenol, 2-methyl-5-amino-4-chlorophenol, 6-methyl-3-amino-2-chlorophenol, 3,4-diaminobenzoic acid, 3-amino-2-methylamino-6-methoxy pyridine and 2,6-dihydroxy-3,4-dimethylpyridine and the physiologically safe salts of these compounds preferably formed with inorganic acids.

6. Preparation as claimed in any of claims 1 to 5, **characterized in that** it contains substantive dyes from the group of nitrophenylenediamines, nitroaminophenols, anthraquinones or indophenols, preferably in a quantity of 0.01 to 20% by weight, based on the colorant as a whole.

7. Preparation as claimed in any of claims 1 to 6, **characterized in that** ammonium or metal salts selected from the group of formates, carbonates, halides, sulfates, butyrates, valerates, caproates, acetates, lactates, glycolates, tartrates, citrates, gluconates, propionates, phosphates and phosphonates of alkali metals, such as potassium, sodium or lithium, alkaline earth metals, such as magnesium, calcium, strontium or barium, or of aluminium, manganese, iron, cobalt, copper or zinc are additionally present.

8. Preparation as claimed in any of claims 1 to 7, **characterized in that** it contains oxidizing agents, more particularly H₂O₂, in a quantity of 0.01 to 6% by weight, based on the solution applied.

9. Preparation as claimed in any of claims 1 to 8, **characterized in that** it contains anionic, zwitterionic or nonionic surfactants.

10. A process for colouring keratin fibres, more particularly human hair, in which a colorant containing
A) at least one N-haloimine corresponding to formula I: 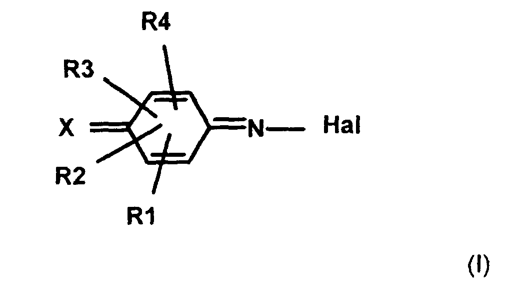 in which R¹, R², R³ and R⁴ represent a hydrogen atom, a halogen atom, a C₁₋₄ alkyl group, a C₁₋₄ alkoxy group; two of the substituents together may even form a fused aromatic or heteroaromatic ring,
X is oxygen or an arylimine and
Hal is fluorine, chlorine, bromine or iodine, and
B) at least one CH-active compound and/or at least one compound from the group consisting of m-phenylenediamine, 2,4-diaminophenoxyethanol, 3-amino-2,4-dichloro-, 2-methyl-5-amino-, 2-methyl-5-(2-hydroxyethylamino)-, 2-methyl-5-amino-4-chloro-, 6-methyl-3-amino-2-chlorophenol, 4-aminobenzoic acid, 3,5-diaminobenzoic acid, 5-aminosalicylic acid, 3-amino-4-hydroxy-, 4-amino-3-hydroxybenzoic acid, 4-aminobenzenesulfonic acid, 3-amino-4-hydroxybenzenesulfonic acid, 4-amino-3-hydroxynaphthalenesulfonic acid, 6-amino-7-hydroxynaphthalene-2-sulfonic acid, 7-amino-4-hydroxynaphthalene-2-sulfonic acid, 4-amino-5-hydroxynaphthalene-2,7-disulfonic acid, 3-amino-2-naphthoic acid, 3-aminophthalic acid, 5-aminoisophthalic acid, 4,5-diaminopyrocatechol sulfate, 3,5-diamino-4-hydroxypyrocatechol sulfate, 4-nitroaniline, 4-nitro-1,3-phenylenediamine, 2-nitro-4-amino-1-(2-hydroxyethylamino)-benzene, 2-nitro-1-amino-4-[bis-(2-hydroxyethyl)-amino]-benzene, 4-amino-2-nitrodiphenylamine-2'-carboxylic acid, 2-amino-6-chloro-4-nitrophenol, 1-amino-5-chloro-4-(2-hydroyethylamino)-2-nitrobenzene, 4,4'-diaminostilbene dihydrochloride, 4,4'-diaminostilbene-2,2'-disulfonic acid, sodium salt, 4,4'-diaminodiphenylmethane, 4,4'-diaminobenzophenone, 3,3',4,4'-tetraaminodiphenyl tetrahydrochloride, 1,3-bis-(2,4-diaminophenoxy)-propane tetrahydrochloride and 1,3-bis-[N-(4-aminophenyl)-2-hydroxyethylamino]-2-propanol,
4-amino-, 2-amino-3-hydroxy-, 3-amino-2-methylamino-6-methoxy-, 2,6-dihydroxy-3,4-dimethylpyridine, 2,3,4-trimethylpyrrole, 2,4-dimethyl-3-ethylpyrrole, 8-aminoquinoline, 6-aminonicotinic acid, 5-aminoisoquinoline, 7-aminobenzimidazole and 2,5-dihydroxy-4-morpholinoaniline and physiologically safe salts of these compounds formed with preferably inorganic acids, 5-methylresorcinol, 2,5-dimethylresorcinol, resorcinol, 3-methoxyphenol, pyrocatechol, hydroquinone, pyrogallol, phloroglucinol, hydroxyhydroquinone, 4-methoxy-, 3-dimethylamino-, 2-(2-hydroxyethyl)-, 3,4-methylenedioxyphenol, 3,4-dihydroxybenzoic acid, gallic acid, 2,4,6-trihydroxybenzoic acid, 4-chlororesorcinol, 1-naphthol, 2,7-dihydroxynaphthalene, 6-dimethylamino-4-hydroxy-2-naphthalene sulfonic acid and 3,6-dihydroxy-2,7-naphthalenesulfonic acid,
and typical cosmetic ingredients is applied to the keratin-containing fibres, left on the fibres for a while, typically for about ca. 30 minutes, and then rinsed out again or washed out with a shampoo.

## Revendications

1. Agent de teinture pour fibres kératiniques, en particulier de cheveux humains, contenant, en tant que composants colorants, au moins une N-halogénoimine de formule I, 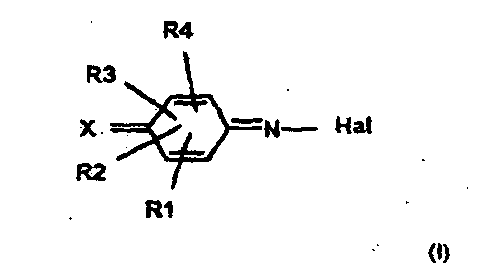 dans laquelle
R¹, R², R³, R⁴ représentent un atome d'hydrogène, un atome d'halogène, un groupe alkyle en C₁-C₄, un groupe alcoxy en C₁-C₄, deux de ces groupes pouvant également former conjointement un cycle aromatique ou hétéroaromatique condensé,
X représente un atome d'oxygène ou une arylimine, et
Hal désigne un atome de fluor, de chlore, de brome ou d'iode
et en plus, au moins un composé choisi parmi au moins un composé ayant un CH actif et/ou au moins un composé compris dans le groupe formé par la m-phénylènediamine, le 2,4-diaminophénoxyéthanol, le 3-amino-2,4-dichloro-phénol, le 2-méthyl-5-aminophénol, le 2-méthyl-5-(2-hydroxyéthylamino)-phénol, le 2-méthyl-5-amino-4-chlorophénol, le 6-méthyl-3-amino-2-chlorophénol, les acides 4-aminobenzoïque, 3,5-diaminobenzoïque, 5-aminosalicylique, 3-amino-4-hydroxybenzoïque, 4-amino-3-hydroxybenzoïque, 4-aminobenzènesulfonique, 3-amino-4-hydroxybenzènesulfonique, 4-amino-3-hydroxynaphtalènesulfonique, 6-amino-7-hydroxynaphtalène-2-sulfonique, 7-amino-4-hydroxynaphtalène-2-sulfonique, 4-amino-5-hydroxynaphtalène-2,7-disulfonique, 3-amino-2-naphtoïque, 3-aminophtalique, 5-aminoisophtalique, le sulfate de 4,5-diamino-pyrocatéchine, le sulfate de 3,5-diamino-4-hydroxypyrocatéchine, la 4-nitroaniline, la 4-nitro-1,3-phénylènediamine, le 2-nitro-4-amino-1-(2-hydroxyéthylamino)-benzène, le 2-nitro-1-amino-4-[bis-(2-hydroxyéthyl)-amino]-benzène, l'acide 4-amino-2-nitrodiphénylamine-2'-carboxylique, le 2-amino-6-chloro-4-nitrophénol, le 1-amino-5-chloro-4-(2-hydroxyéthylamino)-2-nitrobenzène, le dichlorhydrate de 4,4'-diaminostilbène, l'acide 4,4'-diaminostilbène-2,2'-disulfonique, le sel de Na, le 4,4'-diaminodiphénylméthane, la 4,4'-diaminobenzophénone, le tétrahydrochlorhydrate de 3,3',4,4',-tétra-aminodiphényle, le tétrahydrochlorhydrate de 1,3-bis-(2,4-diaminophénoxy)-propane et le 1,3-bis-[N-(4-aminophényl)-2-hydroxyéthylamino]-2-propanol,
la 4-amino-pyridine, la 2-amino-3-hydroxy-pyridine, la 3-amino-2-méthylamino-6-méthoxy-pyridine, la 2,6-dihydroxy-3,4-diméthylpyridine, le 2,3,4-triméthylpyrrol, le 2,4-diméthyl-3-éthylpyrrol, la 8-aminoquinoléine, l'acide 6-aminonicotinique, la 5-aminoisoquinoléine, le 7-aminobenzimidazole et la 2,5-dihydroxy-4-morpholinoaniline, ainsi que les sels de ces composés formés avec, de préférence, des acides inorganiques physiologiquement compatibles,
la 5-méthylrésorcine, la 2,5-diméthylrésorcine, la résorcine, le 3-méthoxyphénol, la pyrocatéchine, l'hydroquinone, le pyrogallol, la phloroglucine, l'hydroxyhydroquinone, le 4-méthoxyphénol, le 3-diméthylaminophénol, le 2-(2-hydroxyéthyl)-phénol, le 3,4-méthylènedioxyphénol, l'acide 3,4-dihydroxy-benzoïque, l'acide gallique, l'acide 2,4,6-trihydroxybenzoïque, la 4-chloro-résorcine, le 1-naphtol, le 2,7-dihydroxynaphtalène, l'acide 6-diméthylamino-4-hydroxy-2-naphtalènesulfonique et l'acide 3,6-dihydroxy-2,7-naphthalène-sulfonique.

2. Agent selon la revendication 1, **caractérisé en ce que**, comme composés de formule I, on met en oeuvre la N-chloro-p-benzoquinone-imine, la 2,6-dichloro-p-benzoquinone-4-chlorimine, la 2,6-dibromo-p-benzoquinone-4-chlorimine, la N-chloro-N'-phényl-p-benzoquinonediimine ainsi que les mélanges quelconque de ces composés.

3. Agent selon l'une quelconque des revendications 1 à 2, **caractérisé en ce que** les N-halogénoimines de formule I sont contenues en une quantité de 0,03 à 65 mmoles, en particulier de 1 à 40 mmoles, pour 100 g de la quantité totale de l'agent colorant.

4. Agent selon l'une quelconque des revendications 1 à 3, **caractérisé en ce que** le composé à CH actif est choisi dans le groupe constitué par l'iodure de 1,2,3,3-tétraméthyl-3H-indolium, le p-toluènesulfonate de 1,2,3,3-tétraméthyl-3H-indolium, le méthanesulfonate de 1,2,3,3-tétraméthyl-3H-indolium, la base de Fischer (1,3,3-triméthyl-2-méthylèneindoline), l'iodure de 2,3-diméthyl-benzothiazolium, le p-toluènesulfonate de 2,3-diméthylbenzothiazolium, la rhodanine, l'acide rhodanine-3-acétique, l'iodure de 1-éthyl-2-quinaldinium, l'iodure de 1,4-diméthylquinolinium, l'iodure de 1-méthyl-2-quinaldinium, l'iodure de 1,4-diméthylquinoléinium, l'acide barbiturique, l'acide thiobarbiturique, l'acide 1,3-diméthylthiobarbiturique, l'acide 1,3-diéthylthiobarbiturique, l'acide diéthylthio-barbiturique, l'oxindol, l'acétate de 3-indoxyle, la coumaranone et la 1-méthyl-3-phényl-2-pyrazolinone.

5. Agent selon l'une quelconque des revendications 1 à 4, **caractérisé en ce que** l'autre composé est choisi dans le groupe constitué par le 2-(2,4-diaminophénoxyéthanol, le 3-amino-2,4-dichlorophénol, le 2-méthyl-5-aminophénol, le 2-méthyl-5-(2-hydroxyéthylamino)-phénol, le 2-méthyl-5-amino-chlorophénol, le 6-méthyl-3-amino-2-chlorophénol, l'acide 3,4-diamino-benzoïque, la 3-amino-2-méthylamino-méthoxypyridine et la 2,6-dihydroxy-3,4-diméthylpyridine, ainsi que les sels de ces composés formés avec, de préférence, des acides inorganiques physiologiquement compatibles.

6. Agent selon l'une quelconque des revendications 1 à 5, **caractérisé en ce qu'**il contient des colorants directs choisis dans le groupe comprenant la nitrophénylènediamine, les nitroaminophénols, l'anthraquinone ou les indophénols, de préférence dans une proportion de 0,01 à 20 % en poids, par rapport à la quantité totale de l'agent colorant.

7. Agent selon l'une quelconque des revendications 1 à 6, **caractérisé en ce qu'**il contient en outre un sel d'ammonium ou de métal choisi dans le groupe des formiates, carbonates, halogénures, sulfates, butyrates, valérates, capronates, acétates, lactates, glycolates, tartrates, citrates, gluconates, propionates, phosphates et phosphonates de métaux alcalins, tels que le potassium, le sodium ou le lithium, de métaux alcalino-terreux, tels que le magnésium, le calcium, le strontium ou le baryum, ou d'aluminium, de manganèse, de fer, de cobalt, de cuivre ou de zinc.

8. Agent selon l'une quelconque des revendications 1 à 7, **caractérisé en ce qu'**il contient un agent oxydant, en particulier H₂O₂, dans une proportion de 0,01 à 6 % en poids, par rapport à la quantité totale de la solution utilisée.

9. Agent selon l'une quelconque des revendications 1 à 8, **caractérisé en ce qu'**il contient des tensioactifs anioniques, zwitterioniques ou non ioniques.

10. Procédé de teinture de fibres kératiniques, en particulier de cheveux humains, dans lequel on applique sur les fibres kératiniques un agent colorant qui contient
A) au moins une N-halogénoimine de formule I, 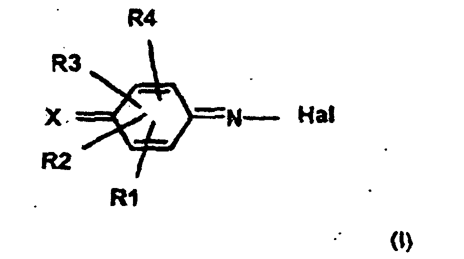 dans laquelle
R¹, R², R³, R⁴ représentent un atome d'hydrogène, un atome d'halogène, un groupe alkyle en C₁-C₄, un groupe alcoxy en C₁-C₄, deux de ces groupes pouvant également former conjointement un cycle aromatique ou hétéroaromatique condensé,
X représente un atome d'oxygène ou une arylimine, et
Hal désigne un atome de fluor, de chlore, de brome ou d'iode, et
B) au moins un composé ayant un CH actif et/ou au moins un composé compris dans le groupe formé par
la m-phénylènediamine, le 2,4-diaminophénoxyéthanol, le 3-amino-2,4-dichloro-phénol, le 2-méthyl-5-aminophénol, le 2-méthyl-5-(2-hydroxyéthylamino)-phénol, le 2-méthyl-5-amino-4-chlorophénol, le 6-méthyl-3-amino-2-chlorophénol, les acides 4-aminobenzoïque, 3,5-diaminobenzoïque, 5-aminosalicylique, 3-amino-4-hydroxybenzoïque, 4-amino-hydroxy-3-benzoïque, 4-aminobenzènesulfonique, 3-amino-4-hydroxybenzènesulfonique, 4-amino-3-hydroxynaphtalènesulfonique, 6-amino-7-hydroxynaphtalène-2-sulfonique, 7-amino-4-hydroxynaphtalène-2-sulfonique, 4-amino-5-hydroxynaphtalène-2,7-disulfonique, 3-amino-2-naphtoïque, 3-aminophtalique, 5-aminoisophtalique, le sulfate de 4,5-diaminopyrocatéchine, le sulfate de 3,5-diamino-4-hydroxypyrocatéchine, la 4-nitroaniline, la 4-nitro-1,3-phénylènediamine, le 2-nitro-4-amino-1-(2-hydroxyéthylamino)-benzène, le 2-nitro-1-amino-4-[bis-(2-hydroxyéthyl)-amino]-benzène, l'acide 4-amino-2-nitrodiphénylamine-2'-carboxylique, le 2-amino-6-chloro-4-nitrophénol, le 1-amino-5-chloro-4-(2-hydroxyéthylamino)-2-nitro-benzène, le dichlorhydrate de 4,4'-diaminostilbène, l'acide 4,4'-diaminostilbène-2,2'-disulfonique, le sel de Na, le 4,4'-diaminodiphénylméthane, la 4,4'-diaminobenzophénone, le tétrahydrochlorhydrate de 3,3',4,4',-tétra-aminodiphényle, le tétrahydrochlorhydrate de 1,3-bis-(2,4-diaminophénoxy)-propane et le 1,3-bis-[N-(4-aminophényl)-2-hydroxyéthylamino]-2-propanol, la 4-amino-pyridine, la 2-amino-3-hydroxy-pyridine, la 3-amino-2-méthylamino-6-méthoxy-pyridine, la 2,6-dihydroxy-3,4-diméthylpyridine, le 2,3,4-triméthylpyrrol, le 2,4-diméthyl-3-éthylpyrrol, la 8-aminoquinoléine, l'acide 6-aminonicotinique, la 5-aminoisoquinoléine, le 7-aminobenzimidazole et la 2,5-dihydroxy-4-morpholinoaniline, ainsi que les sels de ces composés formés avec, de préférence, des acides inorganiques physiologiquement compatibles,
la 5-méthylrésorcine, la 2,5-diméthylrésorcine, la résorcine, le 3-méthoxyphénol, la pyrocatéchine, l'hydroquinone, le pyrogallol, la phloroglucine, l'hydroxyhydroquinone, le 4-méthoxyphénol, le 3-diméthylaminophénol, le 2-(2-hydroxyéthyl)-phénol, le 3,4-méthylènedioxyphénol, l'acide 3,4-dihydroxy-benzoïque, l'acide gallique, l'acide 2,4,6-trihydroxybenzoïque, la 4-chloro-résorcine, le 1-naphtol, le 2,7-dihydroxynaphtalène, l'acide 6-diméthylamino-4-hydroxy-2-naphtalènesulfonique et l'acide 3,6-dihydroxy-2,7-naphthalène-sulfonique,
ainsi que des constituants cosmétiques usuels, on laisse l'agent agir sur les fibres pendant un certain temps, généralement pendant environ 30 minutes, et ensuite, on l'élimine par rinçage, ou par lavage avec un shampooing.
